# NEUE EUROPÄISCHE PATENTSCHRIFT

(11) **EP 2 985 329 B2**
(45) Veröffentlichungstag und Bekanntmachung des Hinweises auf die Entscheidung über den Einspruch: **27.11.2019**
(45) Hinweis auf die Patenterteilung: 17.05.2017
(21) Anmeldenummer: 15179047.4
(22) Anmeldetag: 30.07.2015
(51) Int. Cl.: C09J 7/40, A61L 9/05

(54) **MATERIAL ZUM ABDECKEN VON KLEBENDEN FLÄCHEN**
MATERIAL FOR COVERING ADHESIVE SURFACES
MATERIAU DE RECOUVREMENT DE SURFACES COLLANTES

(30) Priorität: 14.08.2014 DE 102014111629
(43) Veröffentlichungstag der Anmeldung: 17.02.2016
(73) Patentinhaber: Buck Service GmbH, 71083 Herrenberg (DE)
(72) Erfinder: Leipold, Joachim, 72760 Reutlingen (DE); Jaeschke, Edgar, 70794 Filderstadt (DE); Schirmer, Antje, 72108 Rottenburg a.N. (DE); Knapp, Stefan, 68723 Schwetzingen (DE)
(74) Vertreter: Mammel und Maser

(56) Entgegenhaltungen:
- EP-A1- 2 135 518
- EP-A2- 1 471 126
- WO-A1-2008/144123
- WO-A1-2009/095148
- WO-A1-2009/106220
- WO-A1-2011/054434
- WO-A1-2015/007524
- WO-A2-2009/158036
- DE-T2- 69 934 919
- US-A1- 2003 129 343
- "Flockfaser", Wikipedia, 29 March 2013 (2013-03-29), XP055465988,
- "der Stift", Duden, 21 May 2014 (2014-05-21), XP055465998, Retrieved from the Internet: URL:<URL:http://web.archive.org/web/201405 21072843/www.duden.de/rechtschreibung/Stif t_Schreibgeraet_Nagel_Knirps>
- modifizierte Darstellung der Figur 2 aus D1
- Ausdruck 'Pore' www.duden.de über wayback machine vom 13.10.2014

## Beschreibung

Die vorliegende Erfindung betrifft Materialien, die zum Abdecken von klebenden Flächen geeignet sind und die sich anschließend wieder im Wesentlichen rückstandsfrei entfernen lassen.

Insbesondere betrifft die vorliegende Erfindung ein Material zum Abdecken von Oberflächen klebender Sanitärmittel, insbesondere von stückförmigen Toilettenreinigungsmitteln, die auf wenigstens einer Seite wenigstens teilweise mit einer klebenden Paste beschichtet sind.

Seitens der Firma Buck-Chemie GmbH, Herrenberg, wurden klebende Pasten (Haftmittel) entwickelt, mittels denen stückförmige feste Mittel für den Sanitärbereich wie beispielsweise Rimblocks mit einer oder mehreren Phasen, Beduftungstabletten, Entkalkungstabletten oder Reinigungstabletten direkt an der Oberfläche der Toilettenschüssel angeklebt werden können. Die festen stückförmigen Mittel für den Sanitärbereich, wie auch das die Befestigung bewirkende Haftmittel, werden vom Spülstrom nach und nach abgespült, wie in der WO 2009/106220 A1 beschrieben.

Diese Haft- bzw. Klebemittel können Haftvermittler aus der Gruppe der Polyalkylenderivate, der hydrierten Polystyrolderivate, der Silikonsysteme, der Copolymerisate aus der Gruppe der Monoalkylester von Poly/Methyl Vinyl Ethern/Carbonsäureanhydriden, der Olefinhomopolymere und der Copolymere von zwei oder mehr Olefinen, wobei die Olefinhomopolymere und -copolymere auch teilhydriert, teiloxidiert oder über Pfropfmoleküle weiter funktionalisiert sein können, und aus der Gruppe der Polyalkylenimine, auch in alkoxylierter Form, der Polyetheramine (alkoxylierte Amine) und der Polyglycerinpolyetheralkylcarbonsäuren oder der diese Polymergruppen umfassenden Polymere oder Derivate umfassen. Auch hydrophobe Verbindungen wie Öle, Fette oder Wachse, auch teilhydriert, Silikonöle und unpolare Lösemittel sind als Haftvermittler möglich.

Die Viskosität dieser Haftmittel beträgt wenigstens 30 Pa·s, gemessen mit einem Haake-Viskosimeter, System Platte/Platte, Plattendurchmesser 10 mm, bei einem Schergefälle von 2,62 s⁻¹ und 20 °C bzw. zwischen 25 Pa·s und 60 Pa·s gemessen mit einem Rheometer "Brookfield HBDV-III", Helipath Vorsatz und T-E Spindel bei 20 °C.

Diese Haft- oder Klebemittel sind selbst so klebrig, dass sich mit ihnen selbst stückförmige Mittel von 40 g der Abmessung 20 mm x 30 mm x 60 mm mit der Seitenfläche der Abmessung 30 x 60 mm an der vertikalen Oberfläche einer Toilettenschüssel ankleben lassen und dort haften, bis sich das Sanitärmittel nach und nach - im Allgemeinen nach ca. 100 bis 300 Spülungen - aufgelöst hat. Das Verhältnis zwischen Klebemittelmenge und der Masse des stückförmigen Mittels kann zwischen 0,005:1 und 0,2:1, vorzugsweise zwischen 0,02:1 und 0,15:1 und besonders bevorzugt zwischen 0,03:1 und 0,1:1 betragen.

Das Haft- oder Klebemittel selbst wird ebenfalls beim Überspülen mit Wasser nach und nach entfernt, so dass kein unansehnlicher Rest in der Toilettenschüssel verbleibt.

Diese eine Klebeschicht aufweisenden Sanitärmittel könnten sich prinzipiell aus der Verpackung - ohne dass der Verbraucher mit dem Sanitärmittel, der Klebeschicht oder der Toilettenschüssel in Berührung kommt - applizieren lassen.

Problematisch bei der Verpackung solcher klebender Sanitärmittel ist jedoch die Abdeckung der Klebefläche, da übliche Schutzfolien für solche Klebeschichten, wie silikonierte oder fluorierte Folien oder Folien aus Papier, Polyethylen oder Polypropylen, an der Klebeschicht haften und diese beim Ablösen teilweise mit abreißen und so ein wirksames Haften/Kleben des Sanitärmittels an der Toilettenschüssel über die Lebensdauer des Mittels von im Allgemeinen 100 bis 300 Spülungen verhindern.

Um diese auf einer Seite klebenden Sanitärmittel zu bevorraten und auf hygienische Weise applizieren zu können, wurden deshalb spezielle Applikatoren entwickelt, die in der DE 10 2011 001 373 A1 und der DE 10 2009 039 675 A1 beschrieben sind. Diese Applikatoren dienen gleichzeitig als Verpackung. Bei diesen Applikatoren ist das Sanitärmittel mit der Klebeschicht in dem Applikator festgehalten oder verklemmt, derart, dass die Klebeschicht keine Fläche berührt und somit gegen ein unerwünschtes Ankleben an der Verpackung geschützt ist. Während der Applikation wird die Halterung/Verklemmung des Sanitärmittels in dem Applikator gelöst und das Sanitärmittel gegen die Toilettenschüssel gedrückt, um dort zu haften.

Diese Applikatoren bewirken zwar eine hygienische und einfache Applikation und den gewünschten Schutz der Klebeschicht. Nachteilig ist jedoch die relativ aufwändige Herstellung und Form solch eines Applikators für klebende Sanitärmittel und die Gefahr, dass beim Transport durch Bewegung in der Verpackung die Klebeschicht mit Teilen der Verpackung doch in Berührung kommt.

Die Sanitärmittel mit Klebeschicht könnten dann in den üblichen Blisterverpackungen mit Blisterkarte bevorratet und nach Ablösen der Blisterkarte direkt aus der Blisterverpackung - beispielsweise durch Druck auf die Blisterverpackung - in der Toilettenschüssel appliziert werden, wenn ein Material gefunden würde, das die Klebeschicht einerseits schützt, sich andererseits jedoch wieder im Wesentlichen rückstandsfrei von der Klebeschicht des Sanitärmittels vor dem Ankleben entfernen lässt.

Die Aufgabe der vorliegenden Erfindung besteht daher darin, ein Material anzugeben, mittels dessen sich eine haftende/klebende Schicht abdecken und sich dieses Material von der Klebeschicht anschließend wieder im Wesentlichen rückstandsfrei entfernen lässt.

Diese Aufgabe wird durch die Merkmale des Anspruchs 1 gelöst

Wird eine klebende Schicht mit dem erfindungsgemäßen Abdeckmaterial abgedeckt, so liegt das Abdeckmaterial an der klebenden Schicht im Wesentlichen nur mit den Enden der hervorstehenden Elemente an. Die Enden der hervorstehenden Elemente spannen eine Fläche auf, an der der Kontakt mit der Klebeschicht stattfindet. Da die hervorstehenden Elemente eine längliche Form aufweisen, das heißt, die Länge der Elemente größer als deren Durchmesser ist, ist die nach außen weisende Fläche am Ende der hervorstehenden Elemente und somit die Kontaktfläche für jedes hervorstehende Element klein. Nachdem die Enden der Elemente nicht miteinander verbunden sind, muss jeweils beim Abziehen des Abdeckmaterials von der Klebeschicht nur die Adhäsionskraft überwunden werden, die zwischen der Klebeschicht und dem einzelnen Ende wirkt. Nachdem diese aufgrund der kleinen Fläche des Endes der hervorstehenden Elemente gering ist, kann auch die Klebeschicht von einer solchen, durch die Enden der hervorstehenden Elemente aufgespannten Fläche rückstandsfrei entfernt werden.

Bei dem erfindungsgemäßen Abdeckmaterial wirkt die durch die Enden aufgespannte Kontaktfläche somit nicht als (zusammenhängende) Fläche, bei der sich die Adhäsionskräfte aller Enden über die Fläche aufsummieren, sondern nur als eine Vielzahl von einzelnen kleinen Endflächen, wobei in Bezug auf jedes einzelne Ende eines Elements nur eine minimale Adhäsionskraft überwunden werden muss, um die Klebeschicht zu entfernen.

Unter im Wesentlichen rückstandsfrei entfernbar wird im Rahmen der vorliegenden Erfindung verstanden, dass nach dem Ablösen des Abdeckmaterials wenigstens 90 %, vorzugsweise wenigstens 99 % der Masse der Klebeschicht wieder von dem Abdeckmaterial abgelöst werden.

In einer ersten bevorzugten Ausführungsform werden als längliche hervorstehende Elemente Flockfasern eingesetzt. Das Abdeckmaterial ist dann ein beflocktes Trägermaterial, insbesondere ein beflocktes flächiges Trägermaterial. Bei der Beflockung wird Flock, d.h. kurze, nicht zum Verspinnen vorgesehene Textilfasern, auf ein mit Klebstoff beschichtetes Trägermaterial aufgebracht. Unter Textilfasern werden sämtliche Fasern verstanden, die sich textil verarbeiten lassen. Gemeinsam ist ihnen eine im Vergleich zu ihrem Querschnitt große Länge sowie ausreichende Festigkeit und Biegsamkeit. Zu den Textilfasern gehören gemäß Römpp Chemie-Lexikon neben Natur- und Kunstfasern wie Baumwoll-, Kunstseide-, Viscose-, Polyamid-, Polyester- oder Acrylfasern auch Glasfasern, Kohlenstofffasern, Gummifasern oder Metallfasern. Aufgrund ihrer Eigenschaften werden vorzugsweise Polyamidfasern (Nylon, Perlon) als Flockmaterial eingesetzt.

Bei der elektrostatischen Beflockung werden diese Millionen von Fasern in einem elektrischen Feld auf ein mit Klebstoff beschichtetes Trägermaterial aufgebracht. Dabei sorgen die Feldlinien dafür, dass sich alle Fasern senkrecht ausrichten und so eine gleichmäßige, textile Oberfläche erzeugen. Durch die heute verwendeten Klebstoffe sind Beflockungen sehr strapazierfähig und abriebbeständig. Abhängig von Faserstärke und - länge kann entsprechend der gewünschten Funktion, Optik oder Haptik eine samtweiche bis hart-abrasive Oberfläche erstellt werden.

Um ein gutes Ablösen von der Klebeschicht zu erreichen wird der Flock auf dem Trägermaterial erfindungsgemäß gleichmäßig über die gesamte Trägermaterialoberfläche aufgetragen, d.h. es erfolgt Ganzflächenbeflockung. In einer weiteren Variante kann die Beflockung jedoch auch unregelmäßig sein oder ein Muster, eine Herstellerangabe, eine Bildmarke oder einen Markenschriftzug aufweisen.

Infolge des Herstellungsprozesses, d.h. in der Regel der elektrostatischen Beflockung oder der elektrostatisch-pneumatischen Beflockung, sind die Flockfasern senkrecht zur Oberfläche des Trägermaterials ausgerichtet, d.h. die Flockfasern stehen als längliche Elemente von der Trägermaterialoberfläche hervor und die Enden der (gleich lang) geschnittenen kurzen Fasern spannen eine Kontaktfläche auf. Nachdem die Flockfasern senkrecht zur Oberfläche ausgerichtet sind und somit in Art einer Bürste oder eines Nagelbretts parallel zueinander ausgerichtet sind und somit mit ihren Enden, d.h. der Faserquerschnittsfläche, nach außen ragen, findet mit einem jeden hervorstehenden Element jeweils nur eine Wechselwirkung (Adhäsion) über die Faserquerschnittsfläche statt.

Vorzugsweise ist das hervorstehende Element stäbchenförmig, d.h. es weist über seine gesamte Länge einen annähernd gleichen Durchmesser auf, wie es beispielsweise bei Flockfasern der Fall ist.

Die Feinheit der Flockfasern sollte zwischen 0,9 und 200 dtex, und vorzugsweise zwischen 1 und 22 dtex betragen.

Der Durchmesser der Flockfasern beträgt vorzugsweise zwischen 0,5 und 2000 Mikrometer, bevorzugt zwischen 1 und 300 Mikrometer und besonders bevorzugt zwischen 10 und 100 Mikrometer.

Die Länge der Flockfasern kann variieren und hängt selbstverständlich auch von der Steifigkeit der Fasern ab. Vorzugsweise beträgt die Länge der Flockfasern zwischen 0,1 und 10 mm, vorzugsweise zwischen 0,2 und 1,0 mm und besonders bevorzugt zwischen 0,3 und 0,8 mm. Werden jedoch knicksteifere Flockfasern, beispielsweise aus Polyamid, Glas- oder Asbestfasern eingesetzt, können die Flockfasern auch länger sein.

Die mittleren Abstände zwischen den einzelnen Flockfasern liegen in der Regel zwischen 20 Mikrometer und 2000 Mikrometer und vorzugsweise zwischen 40 Mikrometer und 130 Mikrometer. Im Allgemeinen kann jedoch auch bei einem größeren oder geringeren mittleren Abstand zwischen den einzelnen Flockfasern ein gutes Entfernen der Klebeschicht erreicht werden.

Die erreichbare Beflockungsdichte ist umso höher, je kürzer die zur Beflockung eingesetzten Fasern sind. Bei 1 mm langen Fasern beträgt sie vorzugsweise 150 bis 250 Fasern/mm², bei 0,5 mm langen Fasern beträgt sie vorzugsweise 300 bis 400 Fasern/mm².

Damit die Adhäsionskräfte zwischen den hervorstehenden Elementen und der Klebeschicht möglichst gering sind, sollte die Länge eines jeden hervorstehenden Elements wenigstens das Doppelte, vorzugsweise wenigstens das Drei- bis Zehnfache des Durchmessers des Elements betragen.

Nachdem die Klebewirkung der klebenden Schicht flächig ist und sich die Adhäsionskräfte über die mit einem jeden hervorstehenden Element - statistisch - wechselwirkenden Flächenelemente der klebenden Schicht aufsummiert, sind die auf ein jedes hervorstehendes Element wirkenden Adhäsionskräfte umso geringer, je größer die Anzahl der hervorstehenden Elemente pro Fläche des Trägermaterials ist.

Als flächiges Trägermaterial kann beispielsweise Papier, Pappe, eine dünne Platte, eine Folie, Bogenware, eine Metallfolie oder-platte oder auch ein textiles Material eingesetzt werden.

Um die hervorstehenden Elemente auf dem Trägermaterial zu fixieren, weist das Trägermaterial eine Klebstoffschicht aus dem Flockkleber auf, in die die in Richtung des Trägermaterials gerichteten Enden der hervorstehenden Elemente eingebettet sind.

Als Klebstoff kann beispielsweise ein Lösemittel umfassender Klebstoff, ein Dispersionsklebstoff, ein 1- oder 2-Komponentenklebstoff eingesetzt werden, vorzugsweise ein Acryldispersionsklebstoff, ein 2-Komponenten-PUR-Klebstoff oder ein Klebstoff auf der Grundlage von Plastiol, Polyvinylacetat oder Homo- und Copolymeren von Acrylsäureestern. Der Klebstoff (Flocckleber) kann auf das Trägermaterial beispielsweise mittels Siebdruck, Spritzpistole oder einem Tauchbad aufgetragen werden, anschließend wird elektrostatisch beflockt. Je nach verwendetem Flockkleber kann noch eine Wärmenachbehandlung des Klebers und ggfs. ein Reinigungsschritt erfolgen.

Vorzugsweise erfolgt die Beflockung als Flächenbeflockung.

Das flächige Trägermaterial kann auch mehrlagig ausgestaltet sein und auf der Seite, die keine hervorstehenden Elemente aufweist, wenigstens zu 50 % mit adhäsivem Material bestückt sein, das zum Ankleben auf einer weiteren Fläche dient (Etikett).

Nachdem die abzudeckende klebende Schicht hochviskos ist - die Viskosität beträgt im Allgemeinen wenigstens 25 Pa·s gemessen mit dem Rheometer Brookfield HBDV-III, Helipath Vorsatz und T-E-Spindel bei 20 °C - und die hervorstehenden Elemente bzw. Flockfasern dicht gepackt sind, d.h. die Zwischenräume zwischen den hervorstehenden Elementen bzw. Flockfasern sehr klein sind, dringt die Klebemasse nicht in die Zwischenräume zwischen den hervorstehenden Elementen ein sondern berührt vielmehr nur die nach außen weisenden Enden der hervorstehenden Elemente.

In einer weiteren Variante der Erfindung sind die hervorstehenden länglichen Elemente Strukturen, die nach einem Ätzprozess, Lasern oder einem sonstigen partiellen Abtragen von einer Fläche als hervorstehende Elemente stehen bleiben, wie beispielsweise die konischen Strukturen bzw. Mikrostrukturen, die mittels Ablation mit einem Laser aus einem Material wie einem Metall herstellbar sind, wie beispielsweise in der Veröffentlichung von J. Schille, R. Ebert, U. Loeschner, L. Schneider, H. Exner, Hochschule Mittweida in der Veröffentlichung "Material processing with highly repetitive femtosecond laser pulses" über den Vortrag vom 12. - 13. November 2008, 2. ISL Chemnitz, beschrieben.

Zudem können die aus einer Trägermaterialoberfläche hervorstehenden Elemente auch durch in einer Trägermaterialoberfläche eingebundene, hervorstehende röhrenförmige Silikate, Einkristalle, Protein- oder Polymerketten verwirklicht werden, soweit diese als im Wesentlichen längliche Elemente aus einer Trägermaterialoberfläche hervorstehen.

Das erfindungsgemäße Abdeckmaterial lässt sich auch einsetzen, um Flächen anderer klebender Materialien vorübergehend abzudecken, beispielsweise, um die Klebeschicht für ein späteres Ankleben zu schützen.

Ein wesentlicher Einsatzzweck von Materialien zum Abdecken klebender Schichten, die sich wieder im Wesentlichen rückstandsfrei von der klebenden Schicht entfernen lassen, wobei die Materialien ein im Wesentlichen flächiges Trägermaterial sind mit einer Vielzahl auf einer Seite hervorstehender länglicher Elemente, sind klebende Schichten, die einen oder mehrere Haftvermittler aus der Gruppe der Polyalkylenderivate, der hydrierten Polystyrolderivate, der Silikonsysteme, der Copolymerisate aus der Gruppe der Monoalkylester von Poly/Methyl Vinyl Ethern/Carbonsäureanhydriden, der Olefinhomopolymere und der Copolymere von zwei oder mehr Olefinen, wobei die Olefinhomopolymere und -copolymere auch teilhydriert, teiloxidiert oder über Pfropfmoleküle weiter funktionalisiert sein können, und aus der Gruppe der Polyalkylenimine, auch in alkoxylierter Form, der Polyetheramine (alkoxylierte Amine) und der Polyglycerinpolyetheralkylcarbonsäuren oder der diese Polymergruppen umfassenden Polymere oder Derivate oder der hydrophoben Verbindungen wie Öle, Fette oder Wachse, auch teilhydriert, Silikonöle und unpolare Lösemittel umfassen.

Diese klebenden Schichten sind so klebrig, dass sich an ihnen stückförmige Mittel von 40 g an der vertikalen Oberfläche einer Toilettenschüssel ankleben lassen und dort haften.

In einer ersten Variante ist das Sanitärmittel mit der klebenden/haftenden Schicht direkt auf einer beflockten Blisterkarte aufgeklebt. Bei der Entnahme wird die Blisterkarte leicht nach hinten gebogen und so die Beflockung von der Klebeschicht rückstandsfrei entfernt. Anschließend wird das ansonsten noch in der äußeren Blisterverpackung befindliche Mittel mit der Blisterverpackung als Applikator auf der Toilettenschüssel angeklebt und die Blisterverpackung anschließend entsorgt.

In einer weiteren Variante befindet sich das Mittel mit Klebeschicht auf einer beflockten Folie in einer Blisterverpackung, die mit einer Blisterkarte abgedeckt ist. Nach Öffnung der Verpackung wird zunächst die Blisterkarte entfernt, anschließend die mit der Beflockung beschichtete Folie von der Klebeschicht abgezogen und dann das Mittel mit der Blisterverpackung auf der Innenseite der Toilettenschüssel appliziert.

Die Blisterverpackung ist dabei vorzugsweise so ausgestaltet, dass durch einen Druck auf die der offenen Seite gegenüberliegende Seite der Verpackung von außen das Sanitärmittel nun in der Toilettenschüssel mit der Klebeschicht gegen die Wand der Schüssel gedrückt werden kann, wo das Mittel mit der Klebeschicht haftet.

Das erfindungsgemäße Abdeckmaterial eignet sich im Übrigen auch zum Abdecken von Klebeschichten, die nicht vollständig glatt sind.

Es zeigt:
Figur 1: eine Seitenansicht eines erfindungsgemäßen Abdeckmaterials und
Figur 2: ein Sanitärmittel mit Klebebeschichtung, die mit dem erfindungsgemäßen Abdeckmaterial abgedeckt ist in einer Blisterverpackung.

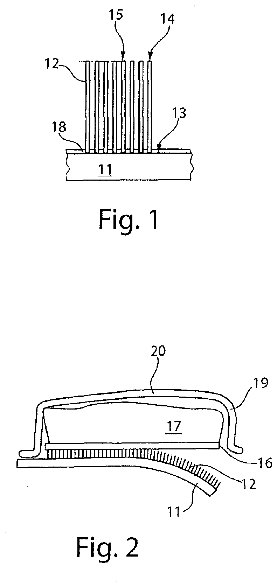

Figur 1 zeigt im Schnitt das flächige Trägermaterial 11, das vorzugsweise eine konstante Schichtdicke aufweisen sollte. Auf dem Trägermaterial 11 befindet sich eine dünne Klebeschicht 18, in die während der vorzugsweise elektrostatischen Beflockung die Flockfasern 12 eingebettet werden.

Die Längsachse der Flockfasern 12 steht senkrecht auf der Oberfläche 13 des Trägermaterials 11, die Längsachsen der Flockfasern 12 verlaufen parallel zueinander. Das dem Trägermaterial 11 entgegengesetzte Ende 14 der Flockfasern 12 steht nach außen, die Endflächen 14 der nahezu gleich langen Flockfasern 12 bilden eine Kontaktfläche 15 aus den nicht miteinander verbundenen Enden 14 der Flockfasern 12 ähnlich der Spitzen der Nägel eines Nagelbretts oder den nach außen weisenden Enden einer Bürste, mit denen die Klebeschicht abgedeckt werden kann. Die Kontaktfläche ist schematisch auf der linken Seite in Figur 1 eingezeichnet.

In Figur 2 ist ein stückförmiges festes Sanitärmittel 17, das an seiner Unterseite die abzudeckende Klebeschicht 16 aufweist, dargestellt. Die Klebeschicht 16 sollte über das gesamte Sanitärmittel 17 etwa dieselbe Dicke aufweisen. Die Schichtdicke der Klebeschicht 16 beträgt im Allgemeinen etwa 0,5 bis 5 mm. Das Sanitärmittel 17 befindet sich in einer Blisterverpackung 19, die das Mittel 17 oben und seitlich abdeckt. Die Klebeschicht 16 des Sanitärmittels 17 ist mit dem erfindungsgemäßen Abdeckmaterial aus Figur 1 im linken Bereich der Figur 2 abgedeckt. Auf der rechten Seite der Figur 2 ist das teilweise von der Klebeschicht 16 rückstandsfrei abgelöste Abdeckmaterial dargestellt.

Nach vollständiger Ablösung des Abdeckmaterials (Figur 2) wird das Mittel 17 in der Blisterverpackung 19 dann mit der Klebeschicht 16 gegen die Innenwand einer Toilettenschüssel gedrückt und durch Druck auf die Oberseite 20 der Blisterverpackung 19 das Mittel 17 mit der Klebeschicht 16 gegen die Wand gedrückt, wo es dann haftet. Anschließend wird die Blisterverpackung 19 entfernt und entsorgt.

## Patentansprüche

1. Verwendung eines Materials zum Abdecken klebender Schichten, wobei das Material ein im Wesentlichen flächiges Trägermaterial (11) ist, das auf seiner einen Seite eine Vielzahl von länglichen Elementen (12) aufweist, die von der Trägermaterialoberfläche (13) hervorstehen und die Enden (14) der hervorstehenden länglichen Elemente (12) nicht miteinander verbunden sind und eine Kontaktfläche (15) aufspannen, die zum Abdecken der Klebeschicht (16) dient.

2. Verwendung nach Anspruch 1, **dadurch gekennzeichnet, dass** die länglichen hervorstehenden Elemente (12) Flockfasern (12) sind.

3. Verwendung nach Anspruch 2, **dadurch gekennzeichnet, dass** die Flockfasern (12) Natur-, Kunst-, Glas-, Kohlenstoff-, Gummi- oder Metallfasern sind.

4. Verwendung nach einem der Ansprüche 2 oder 3, **dadurch gekennzeichnet, dass** die Feinheit der Flockfasern (12) zwischen 0,9 und 200 dtex, und vorzugsweise zwischen 1 und 22 dtex und/oder der Durchmesser der Flockfasern (12) zwischen 0,5 und 2000 Mikrometer, bevorzugt zwischen 1 und 300 Mikrometer und besonders bevorzugt zwischen 10 und 100 Mikrometer und/oder die Länge der Flockfasern (12) zwischen 0,1 mm und 10 mm, vorzugsweise zwischen 0,2 und 1,0 mm und besonders bevorzugt zwischen 0,3 mm und 0,8 mm ist.

5. Verwendung nach einem der vorangegangenen Ansprüche, **dadurch gekennzeichnet, dass** die Länge eines jeden hervorstehenden Elements (12) wenigstens das Doppelte, vorzugsweise wenigstens das Drei- bis Zehnfache des Durchmessers des hervorstehenden Elements ist.

6. Verwendung nach einem der vorangegangenen Ansprüche, **dadurch gekennzeichnet, dass** das flächige Trägermaterial Papier, Pappe, eine dünne Platte, eine Folie, Bogenware, eine Metallfolie oder -platte oder ein textiles Material ist.

7. Verwendung nach einem der vorangegangenen Ansprüche **dadurch gekennzeichnet, dass** die hervorstehenden Elemente (12) an dem Trägermaterial (11) mittels eines Klebstoffs (18) fixiert sind, der ein Lösemittel umfassender Klebstoff, ein Dispersionsklebstoff, ein 1- oder 2-Komponentenklebstoff ist und vorzugsweise ein Acryldispersionsklebstoff, ein 2-Komponenten-PUR-Klebstoff oder ein Klebstoff auf der Grundlage von Plastiolen, Polyvinylacetat oder Homo- und Copolymere von Acrylsäureestern ist.

8. Verwendung nach Anspruch 1, **dadurch gekennzeichnet, dass** die hervorstehenden Elemente (12) Strukturen sind, die durch einen Ätzprozess, Lasern oder ein sonstiges partielles Abtragen von einer Oberfläche (13) als hervorstehende Elemente (12) stehenbleiben.

9. Verwendung nach einem der vorangegangenen Ansprüche, **dadurch gekennzeichnet, dass** die hervorstehenden Elemente (12) in einer Trägermaterialoberfläche (13) eingebundene, hervorstehende, röhrenförmige Silikate, Einkristalle, Protein- oder Polymerketten sind.

10. Verwendung eines Materials nach einem der Ansprüche 1 bis 9 zum Abdecken klebender Schichten, die einen oder mehrere Haftvermittler aus der Gruppe der Polyalkylenderivate, der hydrierten Polystyrolderivate, der Silikonsysteme, der Copolymerisate aus der Gruppe der Monoalkylester von Poly/Methyl Vinyl Ethern/Carbonsäureanhydriden, der Olefinhomopolymere und der Copolymere von zwei oder mehr Olefinen, wobei die Olefinhomopolymere und -copolymere auch teilhydriert, teiloxidiert oder über Pfropfmoleküle weiter funktionalisiert sein können, und aus der Gruppe der Polyalkylenimine, auch in alkoxylierter Form, der Polyetheramine (alkoxylierte Amine) und der Polyglycerinpolyetheralkylcarbonsäuren oder der diese Polymergruppen umfassenden Polymere oder Derivate oder der hydrophoben Verbindungen wie Öle, Fette oder Wachse, auch teilhydriert, Silikonöle und unpolare Lösemittel umfassen.

11. Blisterverpackung umfassend ein stückförmiges Sanitärmittel (17), das eine klebende Schicht (16) aufweist, die mit einem Abdeckmaterial gemäß einem der Ansprüche 1 bis 8 abgedeckt ist.

## Claims

1. Use of a material for covering adhesive layers, wherein the material is an essentially sheetlike supporting material (11) which on one side displays a multiplicity of elongate elements (12) which protrude from the supporting material surface (13) and the ends (14) of the protruding elongate elements (12) are not in connection with each other and stretch out a contact sheet (15) used to cover the adhesive layer (16).

2. Use according to claim 1, **characterized in that** the elongate protruding elements (12) are flock fibres (12) .

3. Use according to Claim 2, **characterized in that** the flock fibres (12) are natural, artificial, glass, carbon, rubber or metal fibres.

4. Use according to either of Claims 2 and 3, **characterized in that** the fineness of the flock fibres (12) is between 0.9 and 200 dtex, and preferably between 1 and 22 dtex
and/or the diameter of the flock fibres (12) is between 0.5 and 2000 micrometres, preferably between 1 and 300 micrometres and more preferably between 10 and 100 micrometres,
and/or the length of the flock fibres (12) is between 0.1 mm and 10 mm, preferably between 0.2 and 1.0 mm and more preferably between 0.3 mm and 0.8 mm.

5. Use according to any preceding claim, **characterized in that** the length of any one protruding element (12) is at least double, preferably at least three to ten times, the diameter of the protruding element.

6. Use according to any preceding claim, **characterized in that** the sheetlike supporting material is paper, paperboard, a thin plate, a film, sheet material, a metal foil or plate or a textile material.

7. Use according to any preceding claim, **characterized in that** the protruding elements (12) are fixed to the supporting material (11) by means of an adhesive (18) which is a solvent-comprising adhesive, a dispersion adhesive, a 1- or 2-component adhesive and preferably an acrylic dispersion adhesive, a 2-component PUR adhesive or an adhesive based on plastisols, polyvinyl acetate or homo- and copolymers of acrylic esters.

8. Use according to Claim 1, **characterized in that** the protruding elements (12) are structures which an etching process, lasering or some other form of partial depletion from a surface (13) has left standing as protruding elements (12).

9. Use according to any preceding claim, **characterized in that** the protruding elements (12) are protruding tubular silicates, single crystals, protein chains or polymer chains bound into a supporting material surface (13).

10. Use of a material according to any one of Claims 1 to 9 for covering adhesive layers comprising one or more adhesion promoters from the group of polyalkylene derivatives, of hydrogenated polystyrene derivatives, of silicone systems, of copolymers from the group of monoalkyl esters of poly/methyl vinyl ethers/carboxylic anhydrides, of olefin homopolymers and of copolymers of two or more olefins wherein the olefin homopolymers and copolymers may also be partially hydrogenated, partially oxidized or, via graft molecules, further functionalized, and from the group of polyalkyleneimines, including in alkoxylated form, of polyether amines (alkoxylated amines) and of polyglycerol polyether alkyl carboxylic acids or of polymers, or derivatives, comprising these polymer groups, or of hydrophobic compounds such as oils, fats or waxes, including partially hydrogenated, silicone oils and apolar solvents.

11. Blister pack comprising a block-shaped sanitary composition (17) having an adhesive layer (16) covered by a covering material according to any one of Claims 1 to 8.

## Revendications

1. Utilisation d'un matériau pour recouvrir des couches adhésives, le matériau étant un matériau support (11) sensiblement plat qui présente, d'un côté, une multitude d'éléments allongés (12) qui dépassent de la surface (13) du matériau support et les extrémités (14) des éléments allongés (12) qui dépassent ne sont pas reliées les unes aux autres et fixant une surface de contact (15) qui sert à recouvrir la couche adhésive (16) .

2. Utilisation selon la revendication 1, **caractérisée en ce que** les éléments allongés (12) qui dépassent sont des fibres de bourre (12).

3. Utilisation selon la revendication 2, **caractérisée en ce que** les fibres de bourre (12) sont des fibres naturelles, synthétiques, de verre, de carbone, de caoutchouc ou métalliques.

4. Utilisation selon l'une quelconque des revendications 2 ou 3, **caractérisée en ce que** la finesse des fibres de bourre (12) se situe entre 0,9 et 200 dtex, et de préférence entre 1 et 22 dtex et/ou le diamètre des fibres de bourre (12) se situe entre 0,5 et 2000 micromètres, de préférence entre 1 et 300 micromètres et de manière particulièrement préférée entre 10 et 100 micromètres et/ou la longueur des fibres de bourre (12) se situe entre 0,1 mm et 10 mm, de préférence entre 0,2 et 1,0 mm et de manière particulièrement préférée entre 0,3 mm et 0,8 mm.

5. Utilisation selon l'une quelconque des revendications précédentes, **caractérisée en ce que** la longueur de chaque élément (12) qui dépasse représente au moins deux fois, de préférence au moins trois fois à dix fois le diamètre de l'élément qui dépasse.

6. Utilisation selon l'une quelconque des revendications précédentes, **caractérisée en ce que** le matériau support plat est en papier, en carton, une plaque mince, un film, un matériau en feuille, un film ou une plaque métallique ou un matériau textile.

7. Utilisation selon l'une quelconque des revendications précédentes, **caractérisée en ce que** les éléments (12) qui dépassent sont fixés au matériau support (11) au moyen d'un adhésif (18), qui est un adhésif comprenant un solvant, un adhésif en dispersion, un adhésif à 1 ou 2 composants et de préférence un adhésif acrylique en dispersion, un adhésif PUR à 2 composants ou un adhésif à base de plastisols, de poly(acétate de vinyle) ou d'homopolymères ou de copolymères d'esters de l'acide acrylique.

8. Utilisation selon la revendication 1, **caractérisée en ce que** les éléments (12) qui dépassent sont des structures qui restent comme éléments (12) qui dépassent par un procédé de gravure, au laser ou par une autre élimination partielle d'une surface (13).

9. Utilisation selon l'une quelconque des revendications précédentes, **caractérisée en ce que** les éléments (12) qui dépassent sont des silicates, des monocristaux, des chaînes protéiniques ou polymères intégrés dans une surface (13) du matériau support, qui dépassent, tubulaires.

10. Utilisation d'un matériau selon l'une quelconque des revendications 1 à 9 pour le recouvrement de couches adhésives, qui comprennent un ou plusieurs promoteurs d'adhérence du groupe des dérivés de polyalkylène, des dérivés hydrogénés de polystyrène, des systèmes siliconés, des copolymères du groupe des esters monoalkyliques de poly/méthylvinyléthers/anhydrides d'acide carboxylique, des homopolymères oléfiniques et des copolymères de deux oléfines ou plus, les homopolymères et les copolymères oléfiniques pouvant également être partiellement hydrogénés, partiellement oxydés ou être fonctionnalisés davantage via des molécules de greffage, et du groupe des polyalkylène-imines, également sous forme alcoxylée, des polyétheramines (amines alcoxylées) et des acides polyglycérolpolyétheralkylcarboxyliques ou des polymères ou des dérivés comprenant ces groupes polymères ou des composés hydrophobes, tels que des huiles, des graisses ou des cires, également partiellement hydrogénées, des huiles siliconées et des solvants non polaires.

11. Emballage blister comprenant un agent sanitaire (17) en forme de morceau, qui présente une couche adhésive (16) qui est recouverte par un matériau de recouvrement selon l'une quelconque des revendications 1 à 8.
